(19) Europäisches Patentamt
European Patent Office
Office européen des brevets

(11) Publication number: 0 216 636
A2

(12) # EUROPEAN PATENT APPLICATION

(21) Application number: 86307324.3

(22) Date of filing: 24.09.86

(51) Int. Cl.4: C 12 P 35/08
C 12 N 1/16
//(C12N1/16,C12R1:88),
(C12P35/08,C12R1:88)

(30) Priority: 24.09.85 JP 211386/85

(43) Date of publication of application:
01.04.87 Bulletin 87/14

(84) Designated Contracting States: DE FR GB IT

(71) Applicant: YAMANOUCHI PHARMACEUTICAL CO. LTD.
No. 5-1 Nihonbashi-Honcho, 2-chome Chuo-ku
Tokyo (JP)

(72) Inventor: Abe, Masami
170 Oaza Takado
Takahagi-shi Ibaraki (JP)

Tamura, Mamoru
170 Oaza Takado
Takahagi-shi Ibaraki (JP)

Eiki, Hideo
283-2 Oaza Takado
Takahagi-shi Ibaraki (JP)

(74) Representative: Geering, Keith Edwin et al
REDDIE & GROSE 16 Theobalds Road
London WC1X 8PL (GB)

(54) **Process for producing oganomycin E.**

(57) A process for producing oganomycin E from cephamycin compound by the action of an esterase is disclosed. The cephamycin for reaction may be provided in situ bacterially. Esterase activity is provided by yeast of genus Torulopsis or by an esterase derived from the yeast. Type strain Torulopsis sp. YE-08907L is described as representative of a yeast having the characteristic enzymic esterase activity.

EP 0 216 636 A2

**Description**

## PROCESS FOR PRODUCING OGANOMYCIN E

The present invention relates to a process for producing 7β-(D-5-amino-5-carboxyvaleramido)-3-hydroxymethyl-7α-methoxy-3-cephem-4-carboxylic acid (I) (hereafter simply referred to as "oganomycin E").

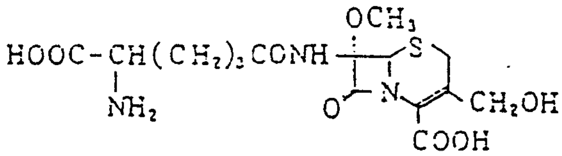
(I)

The present invention provides a process for producing oganomycin E which comprises allowing yeast belonging to the genus Torulopsis, or esterase derived from the yeast to act on cephamycin compound (II) of general formula :

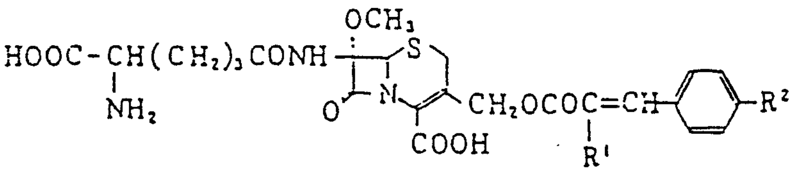
(II)

wherein $R^1$ represents a hydrogen atom or a methoxy group and $R^2$ represents a hydroxy group or a sulfoxy group; and to a process for producing oganomycin E (I) which comprises culturing cephamycin compound (II)-producing bacteria belonging to the genus Streptomyces and allowing yeast belonging to the genus Torulopsis, or esterase derived from the yeast to act on accumulated cephamycin compound (II) in the culture solution to form oganomycin E (I). The invention may provide oganomycin E in salt form; such salts are useful in the field of cephalosporins.

The yeast or esterase derived therefrom may be on a substrate, i.e. bound on a solid carrier.

Known methods for producing oganomycin E by fermentation include culturing Streptomyces chartriusis SF-1623 under aerobic conditions and harvesting this substance from the culture solution (Published Unexamined Japanese Patent Application No.121488/75) and culturing Streptomyces oganonensis and harvesting oganomycin E from the culture solution (Published Unexamined Japanese Patent Application No.43697/82).

However, the former method provides a low yield and is unsuited for industrial production. The latter method gives at least 1000 times the yield but the concentration of oganomycin E accumulated in the culture solution is approximately 5 mg/ml.

As a result of extensive investigations to achieve a higher production concentration, the present inventors have noted that by incorporating yeasts belonging to the genus Torulopsis in some fermentation media and culturing them, accumulated quantities of oganomycin E can be markedly enhanced.

Yeasts belonging to the genus Torulopsis, and esterase derived therefrom, can efficiently hydrolyze the 3-ester of cephamycin compounds (II) to produce oganomycin E (I). Cephamycin compounds (II) may be by-produced during production of oganomycin E by fermentation culturing of oganomycin E-producing bacteria. The present invention can provide an improved yield of oganomycin E by conversion of the cephamycin compounds (II) by-produced by oganomycin E-producing bacteria. The present invention can also provide a process for production of oganomycin E using bacteria capable of producing cephamycin compounds (II).

Examples of oganomycin E-producing bacteria include Streptomyces chartriusis SF-1623 (Published Unexamined Japanese Patent Application No. 121488/75) and Streptomyces oganonensis Y-G19Z (Published Unexamined Japanese Patent Application No.79394/80 and Japanese Patent Application No.110857/85) described above.

Examples of bacteria capable of producing cephamycin compounds (II) include Streptomyces griceus MA-2837 and MA-4125a (Published Unexamined Japanese Patent Application No.3286/71), Streptomyces viridochromogenes, Streptomyces fimbriatus, Streptomyces halstedii, Streptomyces rochei, Streptomyces cinnamonensis and Streptomyces chartrensis (Belgian Patent 764,160). Further the aforesaid Streptomyces oganonensis Y-G19Z by produces cephamycin compounds (II) and thus can also be cephamycin compound-producing bacteria. The cephamycin compounds (II) produced by these microorganisms are specifically cephamycin A ($R^1$ = $-OCH_3$, $R^2$ = $-OSO_3H$), cephamycin B ($R^1$ = $-OCH_3$, $R^2$ = -OH), oganomycin A ($R^1$ = -H, $R^2$ = $-OSO_3H$) and oganomycin B ($R^1$ = -H, $R^2$ = -OH).

The esterase derived from the yeasts belonging to the genus Torulopsis which can be used in the present

invention is an enzyme capable of hydrolyzing the ester bond at the 3-side chain of the aforesaid cephamycin compounds (II). The esterase may be bound to a solid carrier or substrate. A material containing the esterase may be used in the invention; examples of such material are microorganisms carrying the esterase, immobilized esterase etc.

Esterase derived from yeast has scarcely ever been known which can hydrolyze the 3-side chain ester of cephamycin compounds (II). As a result of investigations on various microorganisms isolated from the soil, the present inventors have found a yeast strain having various properties as follows :

(1) Morphological Property

Vegetative cells are spherical or oval, sometimes protractile. The size is 3.0 to 10 μm x 2.0 to 5 μm with oval cells and 3 to 11 μm with spherical ones.

Vegetative propagation occurs by multipolar budding. Pseudomycel, chlamydospore, oidispore, budding spore, etc. formations are absent but a trace of pseudomycel-like formation sometimes occurs. Neither ascospores nor balistospores are formed.

(2) Culturing Properties (cultured at 25°C for 3 to 14 days)

[1] Maltose Medium

The medium is somewhat turbid as a whole and colored pale pink to light orange. Neither skin nor precipitate is formed. Generation of gas is not observed.

[2] Potato Dextrose Agar

Colonies are smooth, glossy, colored pale orange to light orange and rise to a hemispherical shape on agar. No diffusible dye is formed.

(3) Physiological Properties

[1] Fermentation Test :

Fermentation of glucose is weak but positive. Fermentation of fructose, galactose, sucrose, maltose and raffinose is negative.

(2) Utilization of Carbon Source (cultured at 25°C for 21 days)

Maltose -
D-Galactose +
Fructose -
Glucose +
L-Arabinose +
D-Xylose +
Sucrose -
Inositol -
L-Ramonose -
L-Raffinose -
Mannitol -
Lactose -
D-Sorbitol +
Salicine +
Glycerine +
+: utilized
-: not utilized

(3) Assimilation of Nitrates: Positive

(4) Formation of Starch-like Substance:
Negative

(5) Formation of Carotinoid Dye:
Negative (bacterial dye is a non-carotinoidal substance insoluble in acetone and petroleum ether)

(6) Formation of Ester: Positive

(7) Acid-fast: Negative

(8) Decomposition of Oils and Fats: Negative

(9) Vitamin Auxotrophy: None

(10) Growth Temperature:
It grows at 10 to 33° C but does not grow at 5° C and 37° C.

(11) Urease: Positive
In summary, this strain belongs to a non-spored yeast; nutrient cells are spherical or oval; and pseudo-hyphae are sometimes observed as traces; no starch-like substance is formed but assimilation of nitrates is positive; neither skin nor precipitate is formed by liquid culture; colonies are colored pale orange to light orange but this dye is not of carotinoid; further fermentation of glucose is weak but positive. Upon examination of the literature, genera of microorganisms having these properties are the genus Cryptococcus and the genus Torulopsis. In Cryptoccus, cells are covered with a capsule, colonies are viscous and starch-like substance is formed. This strain does not show such properties and hence is distinguishable. The morphological properties, physiological properties, etc. of this strain compare closely with yeasts belonging to the genus Torulopsois. The new strain has characteristic enzymic esterase activity and has been named as the type strain Torulopsis sp. YE-0807L. The type strain has been deposited in the Agency of Industrial Science and Technology, the Fermentation Research Institute under the accession number FERM BP-1158.

The properties of the type strain isolated by the present inventors have been described hereinabove. In addition to this strain and treated products thereof, other yeasts of genus Torulopsis carrying or producing the esterase can also be employed in the present invention.

When selecting a fermentation metabolism system particular attention should be paid to avoiding inhibition of the system and achieving the optimum conditions. This is particularly important in the case of adopting a so-called mixing culture method in which several microorganisms are co-cultured - e.g. cephamycin compound-producing bacteria are cultured in the presence of esterase-producing yeast to produce oganomycin E. Some microorganisms may have a potent productivity of amylases or proteases, they may have a potent productivity of acidic substances or basic substances, they may have a large propagation rate or oxygen absorption rate, etc., and they may disrupt the fermentation metabolism environment and hence reduce the total product yield. In this respect, yeasts are a good group of microorganisms as compared to, e.g. bacteria and molds.

According to the present invention, oganomycin E can be prepared as follows :

Process 1 (enzymatic hydrolysis of cephamycin compounds (II))
This process comprises using cephamycin compound (II) as a substrate, and allowing yeast belonging to the genus Torulopsis, or the esterase produced therefrom, to act thereon to produce oganomycin E. To perform this process, yeast belonging to the genus Torulopsis, or the esterase produced therefrom (which may or may not be carried on a substrate), is mixed with a solution of the cephamycin compound (II); the mixture is shaken at about 30° C at neutral pH and the formed oganomycin E is separated. As the solution of the cephamycin compound (II), there may be used any of a fermentation solution containing the compound (II), a fermentation filtrate and a solution of the compound (II) separated and isolated. As the esterase source, there can be utilized the culture solution per se of the yeast belonging to the genus Torulopsis, cells of the yeast, ground cells, an extract of the esterase active fraction, solid carriers (activated charcoal, diatomaceous earth, hydrophilic gel, high molecular resins, etc.) having immobilized thereon yeast belonging to the genus Torulopsis or esterase derived therefrom.

Process 2 (mixing fermentation method)
In the mixing fermentation method cephamycin compound-producing strain is cultured under culture conditions used for the production of the cephamycin compound by fermentation and yeast belonging to the genus Torulopsis is inoculated and both strains are simultaneously cultured. In general, deep culture using liquid medium is advantageous. In the case of the mixing fermentation method it is preferable that the esterase acts in a bacteria-free environment.

Hereafter the mixing fermentation method will be described in more detail below.

The medium used for the mixing incubation may be any medium as far as it contains nutrient sources that the cephamycin compound-producing bacteria,e.g. those belonging to the genus Streptomyces, can utilize. Namely, synthetic medium, semi-synthetic medium or natural medium can be used. For the composition of such a medium, glucose, sucrose, mannitol, glycerine, dextrin, starch, vegetable oils, etc. are used as carbon sources and as nitrogen sources, meat extract, peptone, gluten meal, cotton seed lees, soybean powders, peanut powders, fish powders, corn steep liquor, dry yeast, yeast extract, ammonium sulfate, ammonium nitrate, urea and other organic or inorganic nitrogen sources are employed. Further metal salts, e.g. sulfates, nitrates, chlorides, carbonates, phosphates, etc. of Na, K, Mg, Ca, Zn, Fe, etc. may be incorporated, if necessary or desired. In particular, the incorporation of magnesium carbonate is effective for increasing the productivity (titer) of the compound (I). Further, if necessary or desired, appropriate antibiotic production-accelerating substances or defoaming agents such as methionine, cystein, cystine, methyl oleate, lard oil, silicone oil, surfactants, etc.may be used.

It is generally advantageous to culture under aerobic conditions. It is desired that the culturing temperature

be in a range of about 18 to about 35° C, preferably about 30° C. Good results are obtained when the pH of the medium is kept in a range of about 5 to about 10, preferably about 6 to about 8. The time period for incubation varies depending upon composition, temperature, etc. of the medium but is generally for about 3 to about 10 days. Inoculation of the yeast belonging to the genus Torulopsis producing the esterase at an initial stage of the incubation is effective. Good results may be obtained when inoculation is at the time of initiating the incubation or up to the second day. In the case of using the esterase instead of the yeast, the esterase may be aseptically incorporated prior to the production of the compound (II); alternatively, the culture solution containing the compound (II) may be cyclized to a container retaining the esterase.

To isolate and harvest the product of the process of the present invention from the culture, there is adopted a conventional method for isolating antibiotics from the culture of microorganisms. The antibiotic (I) is mainly contained in the culture solution and therefore liquid is removed by centrifugation or filtration and thereafter the active substance is extracted from the filtrate. The product can be separated, harvested and purified by means used for the production of ordinary antibiotics utilizing a difference in solubilizing property or solubility in an appropriate solvent, a difference in precipitating property or precipitating rate from a solution, a difference in adsorptive affinity to various adsorbents, a difference in distribution between two liquid phases, etc. These means to be applied singly, in combination of optional orders or repeatedly, if necessary or desired. By oxidative deamination-decarbonation of oganomycin E, oganomycin E is converted into 7β-(4-carboxybutyrlamido)-3-hydroxymethyl-7α-methoxy-3-cephem-4-carboxylic acid (hereafter simply referred to as oganomycin GE) which is then purified and recovered.

In the processes of this invention, the aimed compounds such as oganomycin E (or oganomycin GE) are obtained as a free compound as well as a salt such as a usual alkali metal salt (e.g.,Li-, Na-, Ka- salts),an alkali earth metal salt (e.g., Ca-, Mg-, Ba- salts), an organic amine salt (e.g.,triethylamine salt), etc.

The effect of the present invention lies in that oganomycin E can be efficiently produced by enzymatically hydrolyzing the 3-side chain ester of cephamycin compound (II). Particularly, in the mixing fermentation method, cephamycin compound (II) analogous to oganomycin E that is by-produced in the fermentation medium can be immediately converted into oganomycin E in situ so that culture solution containing oganomycin E in a high concentration can be obtained. According to the process of the present invention, the concentration of oganomycin E produced in the culture solution on the 6th to 7th days during the incubation period may reach 5 times or more that of the conventional process and, the process is more advantageous from an industrial viewpoint.

Next, the process of the present invention will be described in more detail with reference to the examples below.

In these examples, Example 1 relates to production of oganomycin E by acting the esterase and cephamycin B and, Examples 2 and 3 relate to production of oganomycin E by mixing fermentation, and Example 4 relates to utilising a yeast containing esterase.

As a reference example, there is shown a process for producing 7β-(4-carboxybutyrlamido)-3-hydroxymethyl-7α-methoxy-3-cephem-4-carboxylic acid (hereafter simply referred to as oganomycin GE) by oxidative deamination-decarbonation of oganomycin E. Oganomycin GE is a useful compound as an intermediate for producing 7α-methoxycephalosporin antibacterial compounds.

Example 1 (Enzymatic conversion of cephamycin B into oganomycin E)

1) incubation of yeast and preparation of washed cells:

In an Erlenmeyer's flask of 500 ml charged with 60 ml of GPY medium (1.0% glucose, 0.25% peptone and 0.25% yeast extract), Torulopsis sp. YE-0807L strain stored in GPY agar medium was inoculated followed by culturing at 30° C for 3 days at 235 rpm. The culture solution, 5 ml, was transferred to a centrifuge tube. After weighing, the system was subjected to centrifugation at 10000 rpm for 20 minutes and the culture supernatant was separated. The cell portion of the precipitates was redispersed in chilled water followed by centrifugation. After repeating the redispersion and centrifugation twice, chilled water was added up to the initial weight and the cells were diseprsed to obtain a dispersion of the washed cells. The culture solution, culture supernatant and washed cell dispersion were used for oganomycin E production from cephamycin B.

2) Enzymatic reaction

10.0 ml each of a cephamycin B solution (8.92 mM, 0.1M phosphate buffer, pH 7.2) was separately charged in 4 flasks of 200 ml, and the above-described yeast culture solution, culture supernatant and washed cell dispersion as well as water for control were added (2.0 ml each). Each flask was stoppered and the reaction was performed at 30° C with a shaker at 235 rpm. Reaction solution was withdrawn from each flask after 0,7 and 21 hours and the change of the components was examined by high speed liquid chromatography.

3) Results

As shown in Table 1 hereinbelow, in the fractions added with the yeast culture solution and the washed cell diseprsion, cephamycin B was decomposed by 30 to 40% after 7 hours and after 21 hours, by almost 100%; the formation of the corresponding oganomycin E was observed. On the other hand, in the fraction added with the culture supernatant and the control fraction, cephamycin B was decomposed by about 70% after 21 hours but the formation of oganomycin E was merely about 13%.

From the results above, it is noted that the culture solution of Torulopsis sp. YE-0807L used herein has a specific ability of decomposing cephamycin B. This decomposition is a reaction for quantitatively accelerating the formation of oganomycin E and this activity is present in the yeast cells but absent in the culture supernatant.

Table 1

| | Cephamycin B Remained in the Reaction Solution | | | Oganomycin E Produced | | |
|---|---|---|---|---|---|---|
| | 0 Hr. | 7 Hrs. | 21 Hrs. | 0 Hr. | 7 Hrs. | 21 Hrs. |
| Control fraction (water) | 100.0% | 77.0% | 30.0% | 0% | 4.2% | 13.1% |
| Yeast culture solution | 100.0% | 56.8% | 0.0% | 0% | 27.1% | 100.0% |
| Cell dispersion | 100.0% | 59.1% | 1.3% | 0% | 44.1% | 99.1% |
| Culture supernatant | 100.0% | 73.3% | 29.4% | 0% | 3.0% | 12.7% |

Example 2 (Mixing fermentation method)

In a Sakaguchi flask of 500 ml, 100 ml each of seed medium containing 1% starch, 1% glucose, 1.5% soybean powders, 0.5% yeast extract, 0.1% hydrogen disodium phosphate, 0.05% magnesium sulfate and 0.3% sodium chloride was separately charged followed by sterilizing at 120°C for 20 minutes. Streptomyces oganensis Y-19Z was inoculated and cultured at 30°C for 2 days. An Erlenmeymer's flask of 500 ml charged with 50 ml each of a main fermentation medium containing 18% dextrin, 2% glycerine, 3% soybean powders, 2% gluten meal, 0.2% magnesium carbonate and 0.23% sodium hydroxide was prepared. After sterilizing at 120°C for 20 minutes, 2 ml of the above-described seed culture solution was transplanted and, incubation was initiated at 30°C with a rotary shaker at 240 rpm. Separately, a Sakaguchi flask of 500 ml containing 100 ml of GPY medium (1% glucose, 0.25% peptone and 0.25% yeast extract) was sterilized and prepared. Thereafter, esterase-producing yeast Torulopsis sp. YE-0807L (FERM BP-1158) was inoculated followed by seed culturing at 30°C for 2 days. On the first day of the main fermentation of the Y-G19Z strain, 1 ml each of the seed culture solution and the esterase-producing yeast was added and the incubation was continued for 7 days. Subsequent to the second day of the fermentation, 3 flasks were provided for analysis of the concentration of oganomycin E, etc. daily. The concentration of the product was determined by HPLC (column: LS224 (made by Toyo Soda Co., Ltd., 4 mm x 500 mm), eluant: 0.02M citric acid (pH 3.2), detection: UV detector 254 nm).

A linear increase of the concentration of oganomycin E was observed as shown by a mean value of the 3 flasks in Table 2 below. Further the concentration reached 60 mM or more on the 7th day.

Table 2

| Day of Fermentation | 2 | 3 | 4 | 5 | 6 | 7 |
|---|---|---|---|---|---|---|
| Oganomycin E (mg/ml) | 3.7 | 7.3 | 12.1 | 16.5 | 20.7 | 25.5 |

Example 3

In a fermenter of 30 liters, 20 liters of the fermentation medium of Example 2 were sterilized at 121°C for 30 minutes and 0.8 liters of the seed culture solution of the Y-G19Z strain were transplanted. Fermentation was initiated at 30°C. On the first day of the fermentation, 100 ml of the seed culture solution of the esterase-producing yeast in GPY medium was transplanted and mixing culture was performed up to the 7th day. Seed culture of both strains was performed in a manner similar to Example 2.

The concentration of oganomycin E produced changed as shown in Table 3 :

Table 3

| Day of Fermentation | 2 | 3 | 4 | 5 | 6 | 7 |
|---|---|---|---|---|---|---|
| Oganomycin E (mg/ml) | 2.0 | 6.3 | 12.0 | 16.6 | 20.6 | 23.5 |

After completion of the incubation, the pH was adjusted to 4.0 with 4N aqueous hydrochloric acid solution. Diatomaceous earth (Radiolite #600) (trademark) was added to the system followed by filtration. 24 liters of filtrate, including filtration washing liquids,were obtained. This filtrate was passed through a column of 6 liters packed with HP.20 (made by Mitsubishi Chemical Industry Co., Ltd.). The eluate was adjusted to pH 7.0 with a 4N sodium hydroxide aqueous solution. Electrodialysis was performed for 40 hours using an electrodialysis tank using ion exchange (AMV and CMV) made by Asahi Glass Co., Ltd. to effect desalting.

The desalted matter was adsorbed on 5 liters of Dowex 1 x 2 (Cl-) (made by Dow Chemical Co., Ltd.). After washing with water, elution was performed with a 0.2M aqueous salt solution and analysis was performed by HPLC. Fractions of the compound (I) were collected. The eluted fractions were electrically dialyzed for 16 hours using the aforesaid electrodialysis device to effect desalting. After desalting, the system was concentrated under reduced pressure and freeze dried to powder form.

Half of the crude powders were further purified by an avicel column (solvent, isopropanol:water = 7:3) and analysis was performed by HPLC; fractions of the compound (I) showing an elution time of 8 minutes were collected, concentrated under reduced pressure and freeze dried to give 63 g of crude powders (purity of 80%) of the compound (I).

Further for physicochemical analysis a part of the powders (I) was purified by, in sequence, an avicel column (developing solvent, isopropanol:water = 8:2) and a Sephadex G 10 column (developing solvent, water). After freeze drying, drying was performed in vacuum at 50°C for 5 hours to give 200 mg of white powders.

The physicochemical properties of this product were identical with oganomycin E separately prepared.

Reference Example (Purification and separation as oganomycin GE)

A fermentation solution was obtained by performing mixing fermentation in a fermenter of 30 liters in a manner similar to Example 2. To the fermentation solution was added 10% of diatomaceous earth (Radiolite #600). After filtering at pH 4, the pH was once changed to 3.0 and immediately thereafter adjusted to 7.0. At this stage, 500 g of wet cells of Trigonopsis variabilis and 50 ml of a 30% hydrogen peroxide were added to the system. The enzyme reaction was performed at 30°C for 1 hour to give 7β-(4-carboxybutyrylamido)-3-hydroxymethyl-7α-methoxy-3-cephem-4-carboxylic acid (simply referred to as oganomycin GE).

The formation of oganomycin GE and disappearance of oganomycin E were confirmed by comparing the respective standard specimens under the HPCL conditions described above. After completion of the reaction, the pH was again adjusted to 4 followed by centrifugation and filtration. A clear solution was obtained.

The thus obtained oganomycin GE solution was passed through a column (2 liters) packed with HP-21 Resin (trademark, made by Mitsubishi Chemical Industry Co., Ltd.) and then passed through a column (8 liters) of SP-207 Resin (trademark, made by Mitsubishi Chemical Industry Co., Ltd.) to adsorb oganomycin GE thereto. After washing with water, fractions containing the product were collected with 25% acetone water. Then, oganomycin GE was adsorbed to Dowex 1 x 2 (2 liters) column (Cl type). After washing with water, 7 liters of the eluate were obtained using a 0.5M triethylamine hydrochloride solution (pH 6.8). Five liters of the eluate were taken and diluted with water. The solution was then passed through a SP-207 Resin column (4 liters) at pH 3.4 to again adsorb oganomycin GE thereto. After washing with water, elution was performed using 25% acetone water to collect concentrated fractions.

The oganomycin GE solution was adjusted to pH 6.8 with triethyl amine and then concentrated to dryness. Then, the residue was dissolved in 1.5 liters of methylene chloride to remove insoluble matters and 10 g of activated charcoal powder was added thereto. After stirring for 30 minutes, filtration was carried out. The thus obtained clear solution was concentrated to dryness and further dried in vacuum at room temperature for 48 hours to give 113 g of the powdery product. By comparing with data for the standard specimen in spectrometric analyses by NMR, IR, UV, etc. and HPLC as well as TLC, etc., the thus obtained compound was identified to be oganomycin GE triethyl amine salt. Its purity was about 85%.

Example 4 below shows results obtained by utilizing a microorganism (yeast) carrying the esterase of this

invention.

Example 4

By following the same procedure as in Example 1, the culture solution was obtained. The cells in the culture solution were subjected to centrifugation, washed with water, subjected to centrifugation again, and collected to obtain the living-cells solution. Powders of the living-cells were dispersed in 100% cold-alcohol, and, after 30 minutes, the cells were collected, washed with water to obtain a solution containing un-living cells. Each of 5 ml of the living-cells solution or the un-living cells solution was added to a cephamycin B solution (7.3mM, 0.1M phosphoric acid buffer-pH7.2) in a flask, and the mixtures in the flasks were allowed to react by using shaker (30°C, 235 rpm). 0, 4 and 16 hours after the reaction, the reaction solutions were checked by high-performance chromatography. The results are shown as compared with control without yeast cells.

Reaction time & reaction rate

| | 0 | 4 | 16 (hour) |
|---|---|---|---|
| oganomycin formation rate (%) | | | |
| control | 0.0 | 5.8 | 9.6 |
| living-cells | 0.0 | 39.0 | 80.5 |
| un-living cells | 0.0 | 39.4 | 84.2 |
| cephamycin B remain rate (%) | | | |
| control | 100.0 | 93.0 | 39.8 |
| living cells | 100.0 | 57.4 | 7.8 |
| un-living cells | 100.0 | 58.7 | 10.4 |

From the above results, it is apparent that esterase-containing materials (un-living cells as well as living cells) are effective for the reactions of this invention.

## Claims

1. A process for producing 7 -(D-5-amino-5-carboxyvaleramido)-3-hydroxymethyl-7 -methoxy-3-cephem-4-carboxylic acid (I):

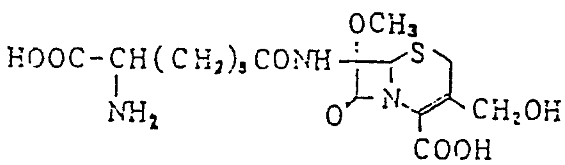

(I)

which comprises allowing yeast belonging to the genus Torulopsis, or esterase derived from said yeast, to act on cephamycin compound represented by general formula (II):

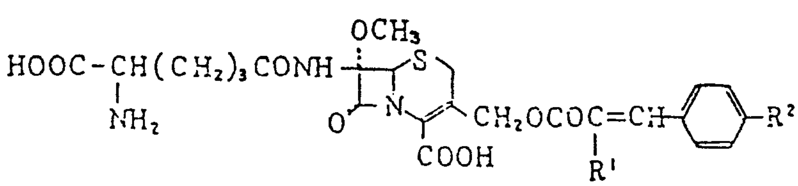

(II)

wherein $R^1$ represents a hydrogen atom or a methoxy group and $R^2$ represents a hydroxy group or a sulfoxy group.

2. A process for producing 7β-(D-5-amino-5-carboxyvaleramido)-3-hydroxymethyl-7α-methoxy-3-cephem-4-carboxylic acid (I):

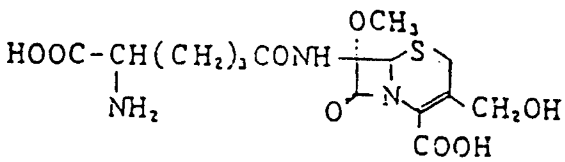

(I)

which comprises culturing bacteria capable of producing cephamycin compound represented by general formula (II):

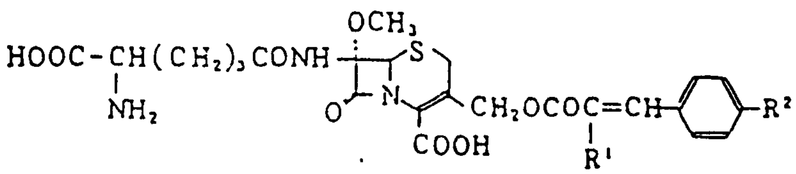

(II)

wherein $R^1$ represents a hydrogen atom or a methoxy group and $R^2$ represents a hydroxy group or a sulfoxy group, which bacteria belongs to the genus Streptomyces, and allowing yeast belonging to the genus Torulopsis, or esterase derived from said yeast, to act on accumulated cephamycin compound in culture solution to form 7β-(D-5-amino-5-carboxyvaleramido)-3-hydroxymethyl-7α-methoxy-3-cephem-4-carboxylic acid, or a salt thereof.

3. The process according to claim 1 or 2 wherein said yeast belonging to the genus Torulopsis is Torulopsis sp. YE-0807L (of which a type strain has been deposited in the Fermentation Research Institute under accession number FERM BP-1158), or a mutant or derivative thereof.

4. A process according to any preceding claim in which the Torulopsis yeast, or esterase derived from said yeast, is bound to a solid carrier.

5. A yeast of genus Torulopsis which is Torulopsis sp. YE-0807L (of which a type strain has been deposited in the Fermentation Research Institute under accession number FERM BP-1158), or a mutant or derivative thereof.

6. A yeast of genus Torulopsis, or esterase derived from said yeast, which has enzymic esterase activity capable of hydrolyzing the ester bond at the 3-side chain of a cephamycin compound of formual II:

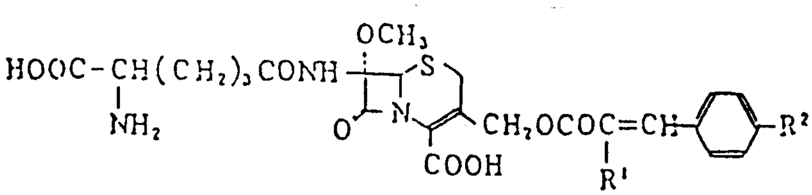

(II)